# DEMANDE DE BREVET EUROPEEN

(11) **EP 4 656 221 A1**
(43) Date de publication de la demande: **03.12.2025**
(21) Numéro de dépôt: 25179490.5
(22) Date de dépôt: 28.05.2025
(51) Int. Cl.: A61M 16/00, A61M 16/04, A61M 16/08, A62B 9/06

(54) **DISPOSITIF DE VENTILATION NON-INVASIVE**

(30) Priorité: 31.05.2024 FR 2405724
(71) Demandeur: Amalric, Caroline Patricia Frédérique Pascaline, 81100 Castres (FR)
(72) Inventeur: Amalric, Caroline Patricia Frédérique Pascaline, 81100 Castres (FR)
(74) Mandataire: Gevers & Orès

(57) **Abrégé**

L'invention concerne un dispositif de ventilation non-invasive (101) comprenant un système (103) de génération d'un flux d'air (F), un conduit tubulaire souple (105), configuré pour guider le flux d'air (F) jusqu'à un orifice (105a) de sortie dudit conduit tubulaire souple (105), un embout buccal (107) comportant une jupe d'étanchéité (107a), configurée pour être positionnée dans une cavité buccale et qui comporte une ouverture (107b) débouchant vers l'extérieur de la cavité buccale dans un conduit de raccordement (107c) et une pièce (109) de raccordement configurée pour former une liaison étanche avec le conduit tubulaire souple (105) d'une part et avec l'embout buccal (107) d'autre part, de sorte qu'une liaison étanche est formée entre le système (103) et la cavité buccale.

## Description

### DOMAINE TECHNIQUE

L'invention est relative au domaine de l'assistance respiratoire. Elle concerne en particulier un dispositif de ventilation non-invasive.

### TECHNIQUE ANTÉRIEURE

Il est connu d'utiliser un dispositif de ventilation mécanique (également appelée ventilation artificielle) pour fournir une assistance respiratoire à une personne souffrant d'insuffisance respiratoire. Un tel dispositif a pour fonction de suppléer ou d'assister la respiration spontanée de la personne qui l'utilise.

La ventilation mécanique est dite invasive lorsque le dispositif de ventilation comprend un élément (par un exemple une sonde endotrachéale ou un tube de trachéotomie) qui pénètre dans les voies respiratoires de l'utilisateur et elle est dite non-invasive lorsque l'élément du dispositif de ventilation servant d'interface avec l'utilisateur (comme par exemple un masque facial ou un masque nasal) ne pénètre pas dans les voies respiratoires de l'utilisateur ou de manière très superficielle au niveau de son nez ou de sa bouche.

La ventilation mécanique non-invasive, appelée par la suite ventilation non-invasive, est notamment utilisée par les personnes souffrant d'insuffisance respiratoire chronique (comme par exemple une personne atteinte de Sclérose Latérale Amyotrophique) qui doivent recourir à un tel dispositif, à domicile, en milieu médicalisé ou de manière ambulatoire (par exemple dans un fauteuil roulant).

Dans ce contexte, le dispositif de ventilation non-invasive doit, dans certains cas, en fonction par exemple du niveau d'atteinte du malade, pouvoir être utilisé jour et nuit de manière continue (i.e. sans interruption). Une utilisation continue du dispositif de ventilation non-invasive implique également de pouvoir l'utiliser lors de la prise d'une douche, y compris lorsque le nez et la bouche de l'utilisateur (et par conséquent l'élément d'interface avec l'utilisateur du dispositif de ventilation non-invasive) sont situés sous un jet d'eau.

Or, tous les dispositifs de ventilation non-invasive connus comprennent un élément d'interface avec l'utilisateur pour lequel l'utilisation sous un jet de douche est problématique. A titre d'exemple, un masque facial comporte une mousse sur son pourtour dont les performances ou l'état peuvent être dégradés lorsqu'elle est mouillée. De même, qu'il s'agisse d'un masque nasal, d'un masque narinaire, d'un masque buccal ou d'un masque facial (qui sont tous classiquement utilisés avec les dispositifs de ventilation non-invasive) leur étanchéité au niveau du contact avec le nez ou la bouche est insuffisante pour une utilisation sans risque sous un jet d'eau. En effet, il est très probable pour ces solutions connues que de l'eau s'infiltre dans le masque et, dans le pire des cas, dans les voies respiratoires de son utilisateur.

Il est donc complexe voire impossible d'utiliser un dispositif de ventilation non-invasive connu lorsque son utilisateur place sa tête sous un jet d'eau ce qui rend son utilisation réellement continue également complexe voire impossible.

### RÉSUMÉ DE L'INVENTION

La présente invention propose une solution à ces inconvénients.

Ainsi, un objectif de l'invention est de proposer un dispositif de ventilation non-invasive capable de fournir une assistance respiratoire jour et nuit, y compris lorsque l'utilisateur du dispositif met sa tête sous un jet de douche et sans que celui-ci risque une infiltration d'eau dans ses voies respiratoires.

À cet effet, l'invention selon un premier aspect a pour objet un dispositif de ventilation non-invasive comprenant :
- un système de génération d'un flux d'air comprenant une entrée d'air, des moyens d'entraînement de l'air et une sortie d'air, ledit système étant configuré pour que de l'air ambiant soit admis au niveau de l'entrée d'air, entraîné par le ventilateur et éjecté au niveau de la sortie d'air sous la forme d'un flux d'air à une pression déterminée ; et,
- un conduit tubulaire souple, raccordé à la sortie d'air du système, et configuré pour guider le flux d'air jusqu'à un orifice de sortie dudit conduit tubulaire souple,

ledit dispositif de ventilation non-invasive étant caractérisé en ce qu'il comprend en outre :
   - un embout buccal comportant une jupe d'étanchéité, configurée pour être positionnée dans une cavité buccale et qui comporte une ouverture débouchant vers l'extérieur de la cavité buccale dans un conduit de raccordement ; et,
   - une pièce de raccordement du conduit tubulaire souple avec l'embout buccal, configurée pour former une connexion fluidique étanche entre le conduit tubulaire souple d'une part et l'embout buccal d'autre part,
de sorte qu'un circuit fluidique étanche est formé entre le système et l'ouverture de l'embout buccal.

Le dispositif de ventilation non-invasive selon l'invention peut comprendre une ou plusieurs des caractéristiques suivantes, prises isolément les unes des autres ou en combinaison les unes avec les autres :
- les moyens d'entraînement de l'air sont un ventilateur.
- le dispositif de ventilation non-invasive comprend en outre un pince nez.
- le conduit tubulaire souple présente un diamètre compris entre 1 et 3 centimètres, de préférence égal à 1,5 ou 2,2 centimètres, et une longueur comprise entre 1 et 4 mètres, de préférence égale à 1,83, 2 ou 3 mètres.
- le système de génération d'un flux d'air est un système portable.
- les dimensions du système sont comprises dans un volume de 30 cm3 et le poids du système est inférieur ou égal à 3 kg.
- l'embout buccal comprend en outre des mors configurés pour permettre le maintien dudit embout buccal dans la cavité buccale par serrage entre une mâchoire supérieure et une mâchoire inférieure.
- la pièce de raccordement est raccordée par emmanchement avec le conduit tubulaire souple, au niveau de l'orifice, et avec l'embout buccal, au niveau du conduit de raccordement.
- la pièce de raccordement est configurée pour se visser sur des filetages complémentaires du conduit tubulaire souple, au niveau de l'orifice, et de l'embout buccal, au niveau du conduit de raccordement.
- la pièce de raccordement comporte des moyens d'évacuation d'un air expiré par un utilisateur dudit dispositif de ventilation non-invasive.

### BRÈVE DESCRIPTION DES DESSINS

L'invention sera mieux comprise à l'aide de la description qui va suivre, donnée uniquement à titre d'exemple et faite en se référant aux dessins annexés dans lesquels :
la figure 1 est une représentation schématique d'un dispositif de ventilation non-invasive selon un mode de réalisation de l'invention ;
la figure 2 est une image d'un ensemble comprenant un conduit tubulaire souple, une pièce de raccordement, un embout buccal et un pince nez selon un mode de réalisation de l'invention ;
la figure 3 est une image d'un embout buccal selon un mode de réalisation de l'invention ; et,
la figure 4 est une image d'une pièce de raccordement selon un mode de réalisation de l'invention.

### DESCRIPTION DES MODES DE RÉALISATION

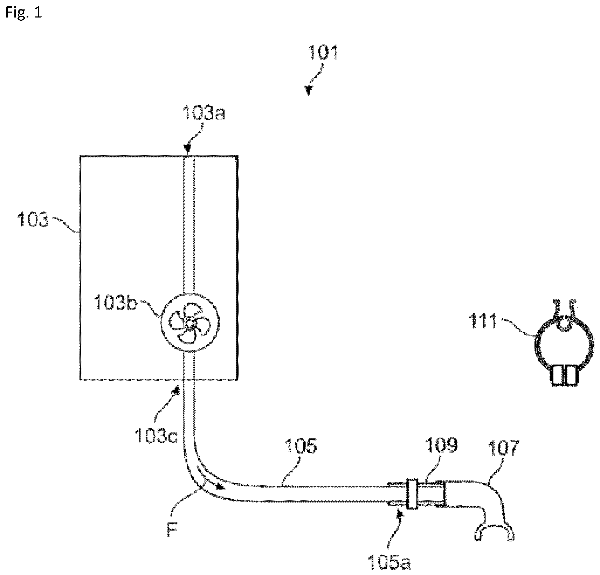

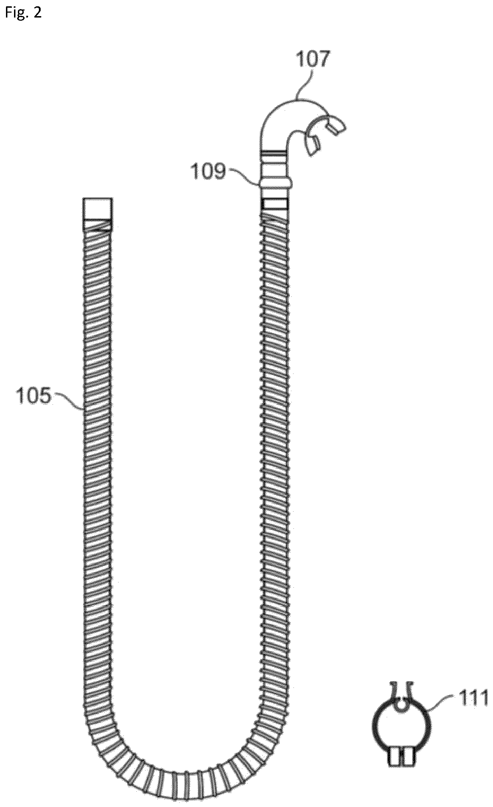

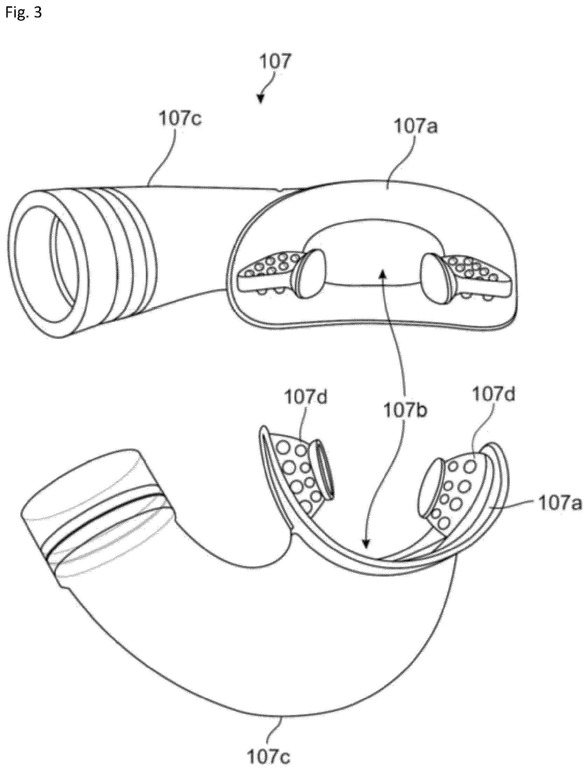

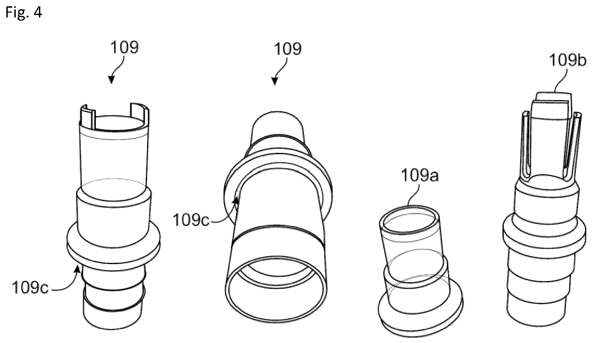

En référence à la figure 1, à la figure 2, à la figure 3 et à la figure 4, nous allons maintenant décrire des modes de réalisation d'un dispositif de ventilation non-invasive selon l'invention.

Le dispositif de ventilation non-invasive 101 comprend un système 103 de génération d'un flux d'air F qui comprend une entrée d'air 103a, un ventilateur 103b et une sortie d'air 103c.

En particulier, un circuit fluidique interne au système 103 relie l'entrée d'air 103a à la sortie d'air 103c en passant par le ventilateur 103b et le système 103 est configuré pour que de l'air ambiant soit admis (aspiré) au niveau de l'entrée d'air 103a, entraîné par le ventilateur 103b et éjecté au niveau de la sortie d'air 103c sous la forme d'un flux d'air F à un débit déterminé. A titre d'exemple, la valeur du débit du flux d'air F est inférieure ou égale à 300 litres par minute. En outre, l'éjection d'air au niveau de la sortie d'air 103c peut être continue ou se faire à intervalle temporel régulier (i.e. selon une fréquence déterminée et pendant une durée déterminée).

Enfin, dans d'autres modes de réalisation, l'entrainement de l'air et son éjection peuvent être réalisés par d'autres moyens d'entrainement de l'air (non-représentés) en lieu et place du ventilateur 103b comme par exemple tout dispositif pneumatique capable de réaliser cette fonction.

Dans différents modes de réalisation également, le système 103 peut également comprendre un filtre (non-représenté) configuré pour filtrer l'air admis (i.e. éliminer les éventuelles impuretés présentes dans l'air ambiant) dans le système 103. En outre, la valeur de débit du flux d'air F généré par le système 103 peut être réglable.

En outre, dans certains modes de réalisation, le système 103 de génération du flux d'air F est un système portable, c'est-à-dire que ces dimensions et son poids sont adaptées pour permettre à un utilisateur de le déplacer simplement, sans l'aide d'une tierce personne ou de moyens supplémentaires.

A titre d'exemple non-limitatif, les dimensions du système 103 peuvent être comprises dans un volume de 30 cm³ et son poids peut être inférieur ou égal à 3 kg.

Le dispositif de ventilation non-invasive 101 comprend également un conduit tubulaire souple 105 qui est raccordé à la sortie d'air 103c du système 103. Le raccord entre le conduit tubulaire souple 105 et la sortie d'air 103c est étanche de sorte que l'intégralité de flux d'air F éjecté du système 103 pénètre dans le conduit tubulaire souple 105. Par exemple, le raccord peut se faire par emmanchement de deux sections cylindriques de diamètres complémentaires (du conduit tubulaire souple 105 et d'une section cylindrique au niveau de la sortie d'air 103c), par vissage de l'un sur l'autre via des filetages complémentaires ou par tout autre moyen permettant la fixation étanche de l'un à l'autre.

En outre, le conduit tubulaire souple 105 est configuré pour guider le flux d'air F jusqu'à un orifice 105a de sortie. Le conduit tubulaire souple 105 permet que le flux d'air F (au niveau de l'orifice 105a) suive les éventuels déplacements d'un utilisateur du dispositif de ventilation non-invasive 101 relativement au système 103.

A titre d'exemples non-limitatifs le conduit tubulaire souple 105 peut présenter un diamètre compris entre 1 et 3 centimètres, et de préférence égal à 1,5 ou 2,2 centimètres, et une longueur comprise entre 1 et 4 mètres, et de préférence égale à 1,83, 2 ou 3 mètres.

Ainsi, il est possible par exemple, lors de la prise d'une douche d'un utilisateur du dispositif 101, de positionner le système 103 à un endroit hors d'atteinte du jet d'eau tout en conservant une certaine mobilité de l'utilisateur.

Le dispositif de ventilation non-invasive 101 comprend encore un embout buccal 107 qui comporte une jupe d'étanchéité 107a qui est configurée pour être positionnée dans une cavité buccale (i.e. l'intérieur d'une bouche) et qui comporte une ouverture 107b débouchant vers l'extérieur de la cavité buccale dans un conduit de raccordement 107c.

Lors de l'utilisation du dispositif, la jupe d'étanchéité 107a est positionnée entre les lèvres et les dents d'un utilisateur et maintenue dans la cavité buccale de sorte que l'air ne peut circuler de l'intérieure de la cavité buccale vers l'extérieur de la cavité buccale, et réciproquement, qu'en passant par l'ouverture 107b et le conduit de raccordement 107c.

Dans l'exemple non-limitatif représenté, l'embout buccal 107 comprend aussi des mors 107d qui sont configurés pour permettre son maintien dans la cavité buccale par serrage entre une mâchoire supérieure et une mâchoire inférieure de l'utilisateur. En l'espèce la jupe d'étanchéité 107a forme un arc de cercle (l'arc de cercle est souple pour pouvoir épouser l'alignement des dents) dont dépasse deux mors 107d, orientés vers l'intérieur de la cavité buccale (i.e. vers le centre du cercle) sensiblement perpendiculairement à la jupe d'étanchéité 107a.

Avantageusement, ces mors 107d permettent un maintien et un retrait facile de l'embout buccal dans la cavité buccale de l'utilisateur.

Le dispositif de ventilation non-invasive 101 comprend enfin une pièce 109 de raccordement du conduit tubulaire souple 105 avec l'embout buccal 107 qui est configurée pour former une connexion fluidique étanche entre le conduit tubulaire souple 105 d'une part et l'embout buccal 107 d'autre part.

Dans l'exemple non-limitatif représenté, la pièce de raccordement 109 est raccordée par emmanchement à la fois avec le conduit tubulaire souple 105 au niveau de l'orifice 105a et avec l'embout buccal 107 au niveau du conduit de raccordement 107c. Plus précisément, le conduit tubulaire souple 105 présente un diamètre inférieur à celui de la pièce de raccordement 109 et s'insère en force dans celle-ci de manière à former une liaison étanche tandis que la pièce de raccordement 109 présente un diamètre inférieure à celui du conduit de raccordement 107c et s'insère en force dans celui-ci de manière à former un liaison étanche.

Dans d'autres modes de réalisation, la pièce de raccordement 109 pourrait être configurée pour se visser sur des filetages complémentaires du conduit tubulaire souple 105, au niveau de l'orifice 105a, et de l'embout buccal 107, au niveau du conduit de raccordement 107c ou pour se fixer au conduit tubulaire souple 105 et à l'embout buccal 107 par tout autre moyen permettant de rendre la liaison étanche. Ainsi, lorsque tous les éléments compris dans le dispositif de ventilation non-invasive sont assemblés, un circuit fluidique étanche (externe au système 103) est formé entre le système 103 et l'ouverture 107b de l'embout buccal 107 (et par conséquent la cavité buccale d'un utilisateur). L'étanchéité dont il est question ici désigne en particulier une étanchéité à l'eau, c'est-à-dire que de l'eau ne peut pas rentrer, depuis l'extérieur, dans le circuit qui relie le système 103 à la cavité buccale d'un utilisateur dans lequel circule de l'air (i.e. le flux d'air F).

Ainsi, avantageusement, grâce à l'invention, il est possible de continuer à bénéficier d'une ventilation non-invasive lorsque la tête d'un utilisateur, et par conséquent, sa bouche et l'embout buccal, sont placés sous un jet de douche sans risquer une infiltration d'eau dans le dispositif et surtout dans les voies respiratoires de l'utilisateur. En outre, dans le mode de réalisation représenté, le dispositif de ventilation non-invasive 101 comprend également un pince nez 111. L'expiration et l'inspiration de l'utilisateur sont alors réalisées uniquement par la bouche, c'est-à-dire par l'intermédiaire de la cavité buccale et à travers l'embout buccal 107, ce qui supprime tout risque de pénétration d'eau dans les voies respiratoires de l'utilisateur via ses narines.

Par ailleurs, dans un mode de réalisation particulier, la pièce de raccordement 109 comporte des moyens 109c d'évacuation d'un air expiré par un utilisateur du dispositif de ventilation non-invasive 101. Par exemple, comme il est visible à la figure 2, la pièce 109 peut comporter un capot 109a s'emboitant autour d'un corps 109b qui présente une forme complémentaire de sorte qu'un canal annulaire 109c (débouchant à l'extérieur de la pièce de raccordement 109) existe entre les deux pour permettre à l'air de circuler depuis l'intérieur de la pièce de raccordement 109 jusque l'extérieur de la pièce de raccordement 109.

En outre, les dimensions du canal annulaire 109c et la forme du capot peuvent être configurés pour que de l'eau n'entre pas par ce canal depuis l'extérieur du dispositif lorsque le dispositif de ventilation non-invasive est positionné sous un jet d'eau. En particulier, en fonctionnement (i.e. lorsqu'une personne utilise le dispositif de ventilation non-invasive 101), la pression de l'air générée dans la pièce de raccordement 109 par la circulation du flux d'air F d'une part, et/ou l'orientation du capot 109a relativement à un éventuel écoulement d'eau d'autre part empêchent l'eau de rentrer dans la pièce de raccordement 109 tout en permettant à de l'air expiré de s'échapper par ce canal. De plus, de l'air expiré par un utilisateur empêche également l'eau de pénétrer dans le dispositif.

Un utilisateur du dispositif de ventilation non invasive peut ainsi expirer de l'air, en cas de fonctionnement comme en cas d'arrêt du système 103, et de l'air peut circuler, si nécessaire, depuis l'intérieur de la cavité buccale jusqu'à l'extérieur de la pièce de raccordement par l'intermédiaire de l'embout buccal et de la pièce de raccordement.

## Revendications

1. Dispositif de ventilation non-invasive (101) comprenant :
- un système (103) de génération d'un flux d'air (F) comprenant une entrée d'air (103a), des moyens (103b) d'entraînement de l'air et une sortie d'air (103c), ledit système (103) étant configuré pour que de l'air ambiant soit admis au niveau de l'entrée d'air (103a), entraîné par le ventilateur (103b) et éjecté au niveau de la sortie d'air (103) sous la forme d'un flux d'air (F) à une pression déterminée ; et,
- un conduit tubulaire souple (105), raccordé à la sortie d'air (103c) du système (103), et configuré pour guider le flux d'air (F) jusqu'à un orifice (105a) de sortie dudit conduit tubulaire souple (105),
ledit dispositif de ventilation non-invasive (101) étant **caractérisé en ce qu'**il comprend en outre :
- un embout buccal (107) comportant une jupe d'étanchéité (107a), configurée pour être positionnée dans une cavité buccale et qui comporte une ouverture (107b) débouchant vers l'extérieur de la cavité buccale dans un conduit de raccordement (107c) ; et,
- une pièce (109) de raccordement du conduit tubulaire souple (105) avec l'embout buccal (107), configurée pour former une connexion fluidique étanche entre le conduit tubulaire souple (105) d'une part et l'embout buccal (107) d'autre part,
de sorte qu'un circuit fluidique étanche est formé entre le système (103) et l'ouverture (107b) de l'embout buccal (107),
et **en ce que** la pièce (109) de raccordement comporte un capot (109a) s'emboitant autour d'un corps (109b) qui présente une forme complémentaire de sorte qu'un canal annulaire (109c), débouchant à l'extérieur de la pièce (109) de raccordement, existe entre les deux pour permettre à l'air de circuler depuis l'intérieur de ladite pièce (109) de raccordement jusque l'extérieur de ladite pièce (109) de raccordement.

2. Dispositif de ventilation non-invasive (101) selon la revendication 1, dans lequel les moyens (103b) d'entraînement de l'air sont un ventilateur.

3. Dispositif de ventilation non-invasive (101) selon la revendication 1 ou la revendication 2, comprenant en outre un pince nez (111).

4. Dispositif de ventilation non-invasive (101) selon l'une quelconque des revendications précédentes, dans lequel le conduit tubulaire souple (105) présente un diamètre compris entre 1 et 3 centimètres, de préférence égal à 1,5 ou 2,2 centimètres, et une longueur comprise entre 1 et 4 mètres, de préférence égale à 1,83, 2 ou 3 mètres.

5. Dispositif de ventilation non-invasive (101) selon l'une quelconque des revendications précédentes, dans lequel le système (103) de génération d'un flux d'air (F) est un système portable.

6. Dispositif de ventilation non-invasive (101) selon la revendication 5, dans lequel les dimensions du système (103) sont comprises dans un volume de 30 cm³ et le poids du système (103) est inférieur ou égal à 3 kg.

7. Dispositif de ventilation non-invasive (101) selon l'une quelconque des revendications précédentes, dans lequel l'embout buccal (107) comprend en outre des mors (107d) configurés pour permettre le maintien dudit embout buccal (107) dans la cavité buccale par serrage entre une mâchoire supérieure et une mâchoire inférieure.

8. Dispositif de ventilation non-invasive (101) selon l'une quelconque des revendications 1 à 7, dans lequel la pièce de raccordement (109) est raccordée par emmanchement avec le conduit tubulaire souple (105), au niveau de l'orifice (105a), et avec l'embout buccal (107), au niveau du conduit de raccordement (107c).

9. Dispositif de ventilation non-invasive (101) selon l'une quelconque des revendications 1 à 7, dans lequel la pièce de raccordement (109) est configurée pour se visser sur des filetages complémentaires du conduit tubulaire souple (105), au niveau de l'orifice (105a), et de l'embout buccal (107), au niveau du conduit de raccordement (107c).
